(19) Europäisches Patentamt
European Patent Office
Office européen des brevets

(11) **EP 3 224 231 B1**

(12) **FASCICULE DE BREVET EUROPEEN**

(45) Date de publication et mention
de la délivrance du brevet:
**06.03.2019 Bulletin 2019/10**

(51) Int Cl.:
*C07C 51/00* (2006.01)        *B01J 23/14* (2006.01)
*B01J 23/18* (2006.01)        *C07C 59/08* (2006.01)

(21) Numéro de dépôt: **11808901.0**

(22) Date de dépôt: **16.12.2011**

(86) Numéro de dépôt international:
**PCT/FR2011/000661**

(87) Numéro de publication internationale:
**WO 2012/085361 (28.06.2012 Gazette 2012/26)**

(54) **PROCÉDÉ DE TRANSFORMATION DE BIOMASSE LIGNOCELLULOSIQUE OU DE CELLULOSE PAR DES ACIDES SOLIDES DE LEWIS NON ZÉOLITHIQUE STABLES À BASE D'ÉTAIN OU D'ANTIMOINE SEUL OU EN MÉLANGE.**

VERFAHREN ZUR UMWANDLUNG VONCELLULOSE- ODER LIGNOZELLULOSE-BIOMASSE MIT STABILEN ZEOLITHFREIEN FESTEN LEWISSÄUREN AUF BASIS VON ZINN ODER ANTIMON ALLEIN ODER ALS MISCHUNG

PROCESS FOR CONVERTING CELLULOSE OR LIGNOCELLULOSIC BIOMASS USING STABLE NON-ZEOLITE SOLID LEWIS ACIDS BASED ON TIN OR ANTIMONY ALONE OR AS A MIXTURE

(84) Etats contractants désignés:
**AL AT BE BG CH CY CZ DE DK EE ES FI GB GR HR HU IE IS IT LI LT LU LV MC MK MT NL NO PL PT RO RS SE SI SK SM TR**

(30) Priorité: **22.12.2010 FR 1005025**

(43) Date de publication de la demande:
**04.10.2017 Bulletin 2017/40**

(73) Titulaire: **Centre National de la Recherche Scientifique**
**75794 Paris Cedex 16 (FR)**

(72) Inventeurs:
• **CHAMBON, Flora**
  **69500 Bron (FR)**
• **ESSAYEM, Nadine**
  **38540 Saint-Just Chaleyssin (FR)**
• **RATABOUL, Franck**
  **69008 Lyon (FR)**
• **PINEL, Catherine**
  **69007 Lyon (FR)**
• **CABIAC, Amandine**
  **69007 Lyon (FR)**
• **GUILLON, Emmanuelle**
  **69390 Vourles (FR)**

(74) Mandataire: **Lavoix**
  **62, rue de Bonnel**
  **69448 Lyon Cedex 03 (FR)**

(56) Documents cités:
• **LINGZHAO KONG ET AL: "Hydrothermal catalytic conversion of biomass for lactic acid production", JOURNAL OF CHEMICAL TECHNOLOGY & BIOTECHNOLOGY, vol. 83, no. 3, 1 mars 2008 (2008-03-01), pages 383-388, XP055003744, ISSN: 0268-2575, DOI: 10.1002/jctb.1797**
• **M. S. HOLM ET AL: "Conversion of Sugars to Lactic Acid Derivatives Using Heterogeneous Zeotype Catalysts", SCIENCE, vol. 328, no. 5978, 30 avril 2010 (2010-04-30), pages 602-605, XP055003736, ISSN: 0036-8075, DOI: 10.1126/science.1183990 cité dans la demande**

**Description**

**DOMAINE DE L'INVENTION**

L'invention concerne un procédé de transformation de biomasse lignocellulosique ou de cellulose en acide lactique mettant en oeuvre des catalyseurs hétérogènes stables à base d'étain et/ou d'antimoine, éventuellement dispersé sur un support. L'utilisation de ces catalyseurs permet d'obtenir directement de l'acide lactique à haute sélectivité tout en limitant la production d'oligosaccharides et de polymères solubles.

**ART ANTÉRIEUR**

Depuis quelques d'années, il existe un vif regain d'intérêt pour l'incorporation de produits d'origine renouvelable au sein des filières carburant et chimie, en complément ou en substitution des produits d'origine fossile. Une voie possible est la conversion de la cellulose, contenue dans la biomasse lignocellulosique, en produits ou intermédiaires chimiques, comme l'acide lactique.

Le terme biomasse lignocellulosique (BLC) ou lignocellulose englobe plusieurs produits présents en quantités variables selon son origine : la cellulose, l'hémicellulose et la lignine. L'hémicellulose et la cellulose constituent la partie carbohydrate de la lignocellulose. Ce sont des polymères de sucres (pentoses et hexoses). La lignine est une macromolécule riche en motifs phénoliques. Par biomasse lignocellulosique, on entend par exemple les produits issus de l'exploitation forestière et les sous-produits issus de l'agriculture comme la paille ainsi que certains végétaux dédiés à haut rendement agricole.

La production de produits chimiques à partir de biomasse lignocellulosique permet à la fois de réduire la dépendance énergétique vis-à-vis du pétrole et de préserver l'environnement à travers la diminution des émissions de gaz à effet de serre sans utiliser de ressources destinées aux usages alimentaires.

La transformation directe de biomasse lignocellulosique ou de cellulose en produits ou intermédiaires chimiques, comme l'acide lactique, est une voie particulièrement intéressante. Par transformation directe, on entend la transformation cellulose - acide lactique sans isoler l'intermédiaire glucose.

L'acide lactique est un acide carboxylique, sa formule chimique est $C_3H_6O_3$ et sa structure se reflète dans son nom systématique, l'acide 2-hydroxy-propanoïque. Comme il possède un carbone asymétrique, il existe deux énantiomères de l'acide lactique. Les applications de l'acide lactique sont principalement celles de son polymère PLA (poly lactic acid) comme conservateur alimentaire mais aussi comme polymères biodégradables, pesticides et herbicides.

La production de l'acide lactique peut se faire par voie chimique ou par voie biologique. Les voies chimiques de production de l'acide lactique connues de l'homme du métier se font via la transformation d'intermédiaires pétrochimiques telles que l'hydrolyse du lactonitrile ou l'hydratation de l'acide propionique. L'acide lactique peut également être produit par fermentation de polysaccharides, qui peuvent être issus de la biomasse, par exemple issus de céréales comme le blé ou le maïs. La demande de brevet EPA-1 953 234 concerne un procédé de production d'acide lactique par fermentation d'un extrait de canne à sucre, au moyen de micro-organisme appartenant aux genres *Bacillus* ou *Sporolactobacillus.*

La valorisation de la biomasse lignocellulosique ou de la cellulose contenue dans la biomasse en catalyse hétérogène est décrite dans la littérature. Par exemple, l'hydrolyse de la cellulose en glucose ou sorbitol en milieu aqueux sur catalyseurs métalliques hétérogènes est décrite dans la demande de brevet EP-A-2 011 569. Rinaldi et al. décrivent la dépolymérisation de la cellulose en milieu liquide ionique en présence de catalyseurs acides de Brønsted (Angew. Chem. Int. Ed., 2008, 47, 8047-8050). Zeng et al. décrivent la conversion de glucose en acide lactique, 5-hydroxy méthylfurfural et acide lévulinique en présence de catalyseurs basiques de types oxydes mixtes Al/Zr (Catal. Lett. (2009) 133 : 221-226). Holm et al. décrivent l'utilisation de catalyseurs hétérogènes acides de Lewis zéolithiques pour transformer le glucose ou fructose en acide lactique (Science (2010), 328, 602-605). Le catalyseur zéolithique acide de Lewis est par exemple la zéolithe Sn-Beta, ou la zéolithe Ti-Beta.

Aussi, l'obtention d'acide lactique par traitement de la cellulose/lignocellulose en conditions hydrothermales en présence de catalyseurs homogènes basiques est connue. Par catalyse homogène basique, Fangmin Jin et Heiji Einomoto (J. Mater. Sci. (2008) 43 : 2463-2471) reportent un rendement de 27% en acide lactique en présence de Ca(OH)$_2$ à une température de 300°C en moins de 5 min de réaction. Kong et al. (J. Chem. Technol. Biotechnol. 83 : 383-388 (2008)) décrivent un procédé hydrothermal de production d'acide lactique à partir de biomasse en milieu eau subcritique en présence de cations de métaux de transition Zn(II), Ni(II), Co(II) and Cr(III)). La demande de brevet WO 03/035582 décrit l'hydrogénolyse du sorbitol à 200°C en utilisant des catalyseurs (Ni, Re)/C qui conduit à des rendements en acide lactique de 5% et de 30% en diols (éthylène glycol et propylène glycol). Shimizu et al. (Green Chem., 2009, 11, 1627-1632) ont montré le rôle primordial de l'acidité de Brønsted sur l'hydrolyse de la cellulose en glucose. Zhang et al. (Angew. Chem. Int. Ed. 2008, 47, 8510-8513) ont étudié la transformation de la cellulose en éthylène glycol et propylène glycol sur des catalyseurs carbures de tungstène/charbon activé avec du nickel comme promoteur (T = 245°C,

P = 6 MPa, H$_2$, eau).

Il n'existe pas de procédé qui permet une transformation directe, c'est-à-dire sans isoler l'intermédiaire glucose, de la cellulose ou plus largement de la biomasse lignocellulosique en acide lactique au moyen de catalyseurs hétérogènes. La demanderesse a découvert un procédé de transformation directe de la cellulose, présente dans la biomasse lignocellulosique, en acide lactique, mettant en oeuvre des catalyseurs hétérogènes stables non zéolithiques à base d'étain ou d'antimoine seul ou en mélange, éventuellement dispersé sur un support. Ce procédé permet d'obtenir un rendement élevé en acide lactique.

**RÉSUMÉ DE L'INVENTION**

L'invention consiste en un procédé de transformation de la biomasse lignocellulosique ou de cellulose en acide lactique en présence d'eau, mettant en oeuvre un catalyseur hétérogène non zéolithique stable à base d'étain et/ou d'antimoine, de préférence dispersé sur un support à base d'oxydes ou un support carboné.

**DESCRIPTION DÉTAILLÉE DE L'INVENTION**

Le procédé de transformation de biomasse cellulosique ou de cellulose selon la présente invention comprend la mise en contact de ladite biomasse ou cellulose en présence d'eau avec un catalyseur hétérogène non zéolithique à base d'étain et/ou antimoine, ledit catalyseur présentant des sites acides de type Lewis.

Ledit catalyseur hétérogène non zéolithique est de préférence à base d'oxyde d'étain et/ou d'antimoine.

De façon préférée, ledit catalyseur est dispersé sur un support à base d'au moins un oxyde ou d'un support carboné

Le procédé permet d'obtenir des conversions élevées du réactif et des sélectivités importantes, en particulier des rendements élevés en acide lactique, tout en limitant la formation d'oligosaccharides ou de polymères hydrosolubles. Ces conversions et sélectivités ne sont obtenues qu'en conditions hydrothermales (présence d'eau) et qu'en présence de catalyseurs non zéolithiques stables à base d'étain et/ou d'antimoine présentant des propriétés acides de type Lewis. En effet, les catalyseurs solides ayant majoritairement une acidité de Brønsted favorisent la production d'oligosaccharides solubles et/ou polymères solubles, représentant une moindre sélectivité en intermédiaires chimiques désirés. Les catalyseurs hétérogènes à base d'étain et/ou d'antimoine, de préférence dispersé sur un support à base d'au moins un oxyde ou un support carboné sont stables dans le milieu réactionnel.

De façon préférée, les supports à base d'oxydes sont choisis parmi les oxyde(s) d'aluminium et/ou de zirconium et/ou de titane et/ou de niobium.

Le catalyseur non zéolithique à base d'étain et/ou d'antimoine, de préférence dispersé sur ledit support présente des sites acides de type Lewis. La teneur en sites acides de type Lewis du catalyseur est de préférence supérieure à 50 % de la teneur totale en sites acides. Sous le terme teneur totale en sites acides, on comprend la somme des sites acides de Lewis et des sites acides de Bronsted.

**La charge**

La biomasse lignocellulosique est essentiellement constituée de trois constituants naturels présents en quantités variables selon son origine : la cellulose, l'hémicellulose et la lignine.

La cellulose (C$_6$H$_{10}$O$_5$)$_n$ représente la majeure partie (50-60%) de la composition de la biomasse lignocellulosique. La cellulose est un homopolymère linéaire semi-cristallin du glucose relié par des liaisons β. La cellulose est insoluble dans l'eau à température et pression ambiantes.

L'hémicellulose est le deuxième carbohydrate en quantité après la cellulose et constitue 20 à 40% en poids de la biomasse lignocellulosique. Contrairement à la cellulose, ce polymère est constitué en majorité de monomères de pentoses (cycles à cinq atomes) et hexoses (cycles à 6 atomes). L'hémicellulose est un hétéropolymère amorphe avec un degré de polymérisation inférieur à celui de la cellulose (30-100), et qui est généralement soluble dans l'eau.

La lignine est une macromolécule amorphe présente dans les composés lignocellulosiques dans des proportions variables selon l'origine du matériau (paille ~ 15%, bois : 20-26%). Sa fonction est le renforcement mécanique, l'hydrophobisation et le soutien des végétaux. Cette macromolécule riche en motifs phénoliques peut être décrite comme résultant de la combinaison de trois unités monomères de type propyl-méthoxy-phénols. Sa masse molaire varie de 5000 g/mol à 10000 g/mol pour les bois durs et atteint 20000 g/mol pour les bois tendres.

La matière première lignocellulosique peut être constituée de bois ou de déchets végétaux. D'autres exemples non limitatifs de matière biomasse lignocellulosique sont les résidus d'exploitation agricole (paille, herbes, tiges, noyaux, coquilles...), les résidus d'exploitation forestière (produits de première éclaircie, écorces, sciures, copeaux, chutes...), les produits d'exploitation forestière, les cultures dédiées (taillis à courte rotation), les résidus de l'industrie agro-alimentaire (résidu de l'industrie du coton, bambou, sisal, banane, maïs, panicum virgatum, alfalfa, noix de coco, bagasse...),

les déchets organiques ménagers, les déchets des installations de transformation du bois, les bois usagés de construction, du papier, recyclé ou non.

La charge utilisée dans le procédé selon l'invention est de la biomasse lignocellulosique ou de la cellulose. La cellulose utilisée peut être cristalline, partiellement amorphe ou amorphe.

La charge biomasse lignocellulosique peut être utilisée sous sa forme brute, c'est-à-dire dans son intégralité, c'est-à dire-contenant ses trois constituants cellulose, hémicellulose et lignine. La biomasse brute se présente généralement sous forme de résidus fibreux ou poudre. En général, elle est broyée pour permettre le transport de celle-ci (déchiquetage).

La charge biomasse lignocellulosique peut aussi être utilisée sous sa forme prétraitée, c'est-à-dire sous une forme contenant au moins une partie cellulosique après extraction de la lignine et/ou de l'hémicellulose.

La biomasse subit de préférence un prétraitement afin d'augmenter la réactivité et l'accessibilité de la cellulose au sein de la biomasse avant sa transformation. Ces prétraitements sont de nature mécanique, thermochimique, thermo-mécanico-chimique et/ou biochimique et provoquent la décristallinisation partielle ou totale de la cellulose, la solubilisation totale ou en partie de l'hémicellulose et/ou de la lignine ou l'hydrolyse partielle de l'hémicellulose suivant le traitement.

Les traitements mécaniques vont au delà du simple déchiquetage car ils modifient de la structure chimique des constituants. Ils améliorent l'accessibilité et la réactivité de la cellulose par sa décristallinisation partielle ou totale et par l'augmentation de la surface d'échange. Les traitements mécaniques incluent la réduction de la taille des fibres ou particules élémentaires, par exemple par mise en copeaux de la biomasse à l'aide d'une coupeuse, par broyage de la biomasse (ajustement de la granulométrie), déstructuration des copeaux sur presse ou défibrage par abrasion des copeaux, après préchauffage. Les traitements mécaniques peuvent être opérés en mode décentralisé près de la production de la biomasse ou en mode centralisé alimentant directement la transformation.

Les traitements thermochimiques incluent la cuisson de la biomasse à haute température (150-170°C) en milieu acide dilué (principalement acide sulfurique, mais aussi acide phosphorique, acide acétique ou acide formique), en milieu alcalin (soude, sulfites, chaux...) ou en milieu oxydant (oxydation humide à l'air ou à l'oxygène ; peroxyde en milieu alcalin ; acide peracétique). Les autres traitements thermochimiques incluent des traitements aux solvants (éthanol à chaud) ou la torréfaction qui peut se définir comme une pyrolyse à température modérée et temps de séjour contrôlé car elle s'accompagne d'une destruction partielle de la matière lignocellulosique. Les technologies connues pour la torréfaction sont par exemple le four tournant, le lit mobile, le lit fluidisé, la vis sans fin chauffée, le contact avec de billes métalliques apportant la chaleur. Ces technologies peuvent éventuellement utiliser un gaz circulant à co ou contre courant comme de l'azote ou tout autre gaz inerte dans les conditions de la réaction.

Les traitements thermo-mécanico-chimiques incluent les traitements à la vapeur (explosion à la vapeur appelés encore hydrolyse flash ou "steam-explosion"), le traitement AFEX (ammonia fiber explosion) à l'ammoniaque ou l'extrusion bi-vis avec des réactifs chimiques divers.

Le prétraitement permet de préparer la biomasse lignocellulosique en séparant la partie carbohydrate de la lignine et ajustant la taille des particules de biomasse à traiter. La taille des particules de biomasse après le prétraitement est généralement inférieure à 5 mm, de préférence inférieure à 500 microns.

**Le catalyseur**

Les catalyseurs utilisés pour la transformation de la biomasse lignocellulosique ou de la cellulose sont à base d'étain et/ou d'antimoine, de préférence dispersé à la surface d'un support, de préférence un support à base d'oxydes choisi parmi les oxyde(s) d'aluminium et/ou de zirconium et/ou de titane et/ou de niobium ou un support carboné, lesdits catalyseurs présentant des sites acides de type Lewis.

L'acidité d'un catalyseur est la résultante de deux types d'acidité combinés : une acidité de Lewis, caractérisée par la présence d'une lacune électronique sur un atome, et une acidité de Brønsted, caractérisée par une aptitude à céder un proton. La nature des sites acides peut se caractériser par adsorption de pyridine suivie par spectroscopie IR conformément à la méthode décrite dans *[M. Guisnet, P. Ayrault, C. Coutanceau, M.F. Alvarez, J. Datka, J. Chem. Soc., Faraday Trans. 93, 1661 (1997)]*.

Les solides selon l'invention sont caractérisés par des propriétés acides superficielles majoritairement de type Lewis, de préférence la teneur en site acides de Lewis est supérieure à 50 % de la teneur totale en sites acides. Les sites acides de type Lewis sont associés à la présence d'espèces d'étain et/ou d'antimoine coordinativement insaturées mais aussi aux espèces caractéristiques du support comme par exemple, dans le cas d'une support à base d'oxyde $Al^{3+}$, $Zr^{4+}$, $Ti^{4+}$, $Nb^{5+}$. Il est connu que la coordination des espèces d'étain et/ou d'antimoine dépendent de leur dispersion, de la teneur en Sn ou en Sb, de la nature des précurseurs et des traitements thermiques. L'acidité de Lewis peut être caractérisée par exemple par spectroscopie IR.

Lorsque le support utilisé pour disperser de l'étain et/ou de l'antimoine est un oxyde, il comprend au moins un oxyde, choisi de préférence parmi l'alumine, la zircone, l'oxyde de niobium, l'oxyde de titane, la silice, les alumino-phosphates, les composés mésostructurés seuls ou en mélange préparés selon toute technique connue de l'Homme du métier. Par exemple, les supports peuvent être synthétisés par précipitation, synthèse sol-gel suivi d'un traitement thermique. Les solides obtenus présentent l'avantage d'être mésoporeux et stables thermiquement et en conditions hydrothermales.

Le support peut également être un support carboné comme par exemple des charbons actifs, du noir de carbone, des solides microporeux ou mésoporeux carbonés comme par exemple les nanotubes de carbone, les fibres de carbone. Les supports carbonés sont préparés selon toute technique connue de l'Homme du métier. Les supports carbonés peuvent subir un traitement afin de modifier par exemple leurs propriétés d'acidité, d'hydrophobicité et de texture. Citons par exemple, les traitements thermiques, oxydants, réducteurs.

De manière préférée, les supports sont à base d'oxyde(s).

La teneur en étain et/ou antimoine est comprise entre 1 à 100% en poids, de préférence entre 1 et 50% poids, de préférence entre 1 et 30%, et encore plus préférentiellement entre 1 et 20% poids, les pourcentages étant exprimés en % poids de métal par rapport à la masse totale du catalyseur.

Les précurseurs d'étain ou d'antimoine sont choisis parmi les hydrures, les halogénures, les oxydes, les sulfures, ou des composés organométalliques respectivement d'étain ou d'antimoine.

On citera par exemple les hydrures d'étain, les halogénures d'étain (chlorures, bromures, iodures, fluorures d'étain), les oxydes d'étain, les sulfures d'étain. Les chlorures d'étain sont les précurseurs usuels. L'utilisation de chlorure d'étain en solution dans de l'acide chlorhydrique est préférée. Les précurseurs d'étain peuvent être des organométalliques comme par exemple les composés alkyl étain tel que le tétrabutylétain.

Pour les précurseurs d'antimoine, on citera les hydrures d'antimoine, les halogénures d'antimoine (chlorures, les iodures, les fluorures d'antimoine), les oxydes d'antimoine, les sulfures d'antimoine. Les précurseurs d'antimoine peuvent également être des composés organométalliques d'antimoine.

La préparation des catalyseurs hétérogènes stables à base d'étain ou d'antimoine seul ou en mélange, de préférence dispersé sur le support à base d'oxydes ou le support carboné est réalisée par toute méthode connue de l'homme du métier.

Une méthode de préparation consiste en une imprégnation d'une solution d'acide stannique et/ou d'oxyde d'antimoine et l'hydroxyde de zirconium et/ou de titane et/ou d'aluminium et/ou de niobium, éventuellement suivie d'un séchage.

La présence d'étain et/ou d'antimoine sur le support à base d'oxydes entraîne la formation d'oxyde d'étain et/ou d'oxyde d'antimoine.

Le catalyseur utilisé dans la présente invention à base d'étain et/ou d'antimoine, de préférence dispersé sur un support à base d'oxydes choisi parmi les oxyde(s) d'aluminium et/ou de zirconium et/ou de titane et/ou de niobium ou un support carboné peut subir à l'issue de sa préparation ou au cours de sa préparation un traitement thermique. Ledit traitement thermique est avantageusement réalisé entre 300°C et 1000°C. Il peut être réalisé sous air, sous atmosphère réductrice telle que l'hydrogène, sous azote, pur ou en mélange.

Ledit traitement thermique du catalyseur peut réalisé à toute étape de sa préparation, avant ou après l'étape de mise en forme.

Le catalyseur ainsi obtenu est stable, thermiquement et en conditions hydrothermales.

Le catalyseur utilisé dans la présente invention peut contenir un liant.

Le catalyseur utilisé dans la présente invention peut être sous forme de poudre, d'extrudés, de billes ou de pastilles.

Le catalyseur utilisé selon l'invention est stable et régénérable, c'est-à-dire qu'il ne subit pas de lixiviation pendant la réaction. A l'issue d'une étape de lavage ou de combustion des espèces hydrocarbonées déposées sur le catalyseur après réaction, le catalyseur présente les mêmes performances catalytiques initiales.

**Procédé de transformation**

Le procédé de transformation de la biomasse lignocellulosique ou de la cellulose selon l'invention comprend la réaction dans un milieu contenant de l'eau en présence de la composition catalytique conforme à l'invention.

Par milieu contenant de l'eau on désigne les milieux liquides conventionnels comme les alcools, tels que le méthanol ou l'éthanol, et l'eau, et les milieux non conventionnels comme les liquides ioniques ou les milieux supercritiques de densité type liquide.

La teneur massique en eau dans le milieu est généralement supérieure à 1%. Le milieu peut aussi consister entièrement d'eau. De préférence, le milieu est de l'eau.

Ce procédé est peut être effectué en présence d'un gaz choisi parmi l'air, un gaz neutre ($N_2$, He, Ar...) ou un gaz réducteur comme l'hydrogène.
Le procédé est opéré à des températures comprises entre 160°C et 250°C, de préférence entre 175 et 250°C, et à une pression comprise entre 0,5 et 20 MPa, de préférence entre 2 et 10 MPa.

La réaction peut être opérée selon différents modes de réalisation. Ainsi, la réaction peut être mise en oeuvre en discontinu ou en continu, par exemple en lit fixe. On peut opérer en réacteur fermé ou semi-ouvert.

Le catalyseur est introduit dans le procédé à raison d'une quantité correspondant à un rapport massique biomasse/catalyseur compris entre 1 et 1000, de préférence entre 1 et 500, de préférence entre 1 et 100, de préférence entre 1 et 50 et encore préférentiellement entre 1 et 25.

La biomasse est introduite dans le procédé à raison d'une quantité correspondant à un rapport massique (milieu contenant de l'eau)/biomasse compris entre 1 et 1000, de préférence entre 1 et 500 et encore préférentiellement entre 5 et 100. Le taux de dilution de la biomasse dans le milieu aqueux est donc entre 1:1 et 1:1000, de préférence entre 1:1 et 1:500 et encore préférentiellement entre 1:5 et 1:100.

Si l'on choisit un procédé en continu, la vitesse massique horaire (débit de charge massique/masse de catalyseur) est entre 0,01 et 5 $h^{-1}$, de préférence entre 0,02 et 2 $h^{-1}$.

**Les produits obtenus et leur mode d'analyse**

Après la réaction, le milieu réactionnel est prélevé et centrifugé. Le liquide réactionnel est ensuite analysé par chromatographie liquide haute performance (HPLC) en utilisant la réfractométrie pour déterminer la teneur en produits de conversion de la solution aqueuse.

Les produits de la réaction sont solubles dans l'eau. Ils sont constitués de monosaccharides et de leurs dérivés, d'oligosaccharides, mais aussi de polymères solubles formés par combinaisons successives des dérivés des monosaccharides.

Par monosaccharides, on désigne les sucres simples (hexoses, pentoses) produits par dépolymérisation complète de la cellulose et/ou hémicellulose, en particulier, le glucose, le mannose, le xylose, le fructose...

Par dérivés des monosaccharides on désigne les produits pouvant être obtenus par déshydratation, isomérisation, réduction ou oxydation :

- des sucres alcools, des alcools et polyols : en particulier le sorbitol, le xylitol, le glycérol, l'éthylène glycol, le propylène glycol, l'éthanol, le méthylbutane diol...
- des cétones, des hexane-diones telles que la 2,5-hexanedione...
- des acides carboxyliques et leurs esters, des lactones : l'acide formique, acide lévulinique, lévulinates d'alkyles, acide lactique, lactique d'alkyles, acide glutarique, glutarates d'alkyles, l'acide 3-hydroxypropanoique, la 3-hydroxybutyrolactone, la $\gamma$-butyrolactone,
- des éthers cycliques tel que le tétrahydrofurane (THF), le méthyltétrahydrofurane (Me-THF), le furane dicarboxylique acide, le 5-(hydroxyméthyle)furfural.

Par oligosaccharides, on désigne un carbohydrate ayant pour composition $(C_6H_{10}O_5)_n$ où n est supérieur à 1 obtenus par hydrolyse partielle de la cellulose, ou de l'hémicellulose, ou de l'amidon.

Par polymères solubles, on désigne tous les produits issus de la condensation entre monosaccharides, oligosaccharides et/ou dérivés des monosaccharides.

La quantité des produits de réaction solubles dans l'eau (monosaccharides et dérivés, oligosaccharides, polymères solubles) est déterminée par l'analyse COT (Carbone Organique Total) qui consiste en la mesure du carbone en solution. La quantité des monosaccharides et leurs dérivés est déterminée par analyses HPLC.

La conversion (équivalent au % de solubilisation) de la biomasse ou de la cellulose est calculée suivant l'équation suivante :

$$C = 100 * C_{solubilisé} / C_{initial}$$

dans laquelle $C_{solubilisé}$ représente la quantité de carbone solubilisé analysée par COT (mg) et $C_{initial}$ la quantité de carbone en début de réaction contenue dans la biomasse ou cellulose solide.

Les rendements molaires en dérivés du glucose sont calculés au moyen de l'analyse HPLC. Chaque composé est corrigé du nombre d'atome de carbone contenu dans l'unité glucose.

Les rendements molaires en un dérivé i sont calculés comme suit :

$$Rdti = 100 * (nC_{Pi}/6) * (P_i/Glu_0)$$

où $nC_{pi}$ représente le nombre d'atome de carbones du dérivé i, Pi le nombre de moles du produit $P_i$ et $Glu_0$ le nombre de moles d'unités glucose contenues dans la biomasse ou la cellulose en début de réaction.

La formation d'oligosaccharides et de polymères solubles correspondent à une perte de carbone. Cette perte de carbone est déduite des analyses COT et HPLC. Le rendement en oligosaccharides et polymères solubles est calculé selon l'équation suivante :

$$Rdt_{olig} = C - \sum rdt_i$$

où C représente la conversion de la cellulose et $\sum rdt_i$ la somme des rendements molaires de tous les monosaccharides et leurs dérivés analysés par HPLC.

## EXEMPLES

Exemple 1 : Préparation du catalyseur C1 non zéolithique à base d'oxyde d'étain dispersé sur un support à base d'oxyde d'alumine (conforme à l'invention)

Le catalyseur est préparé en utilisant comme matière première de l'hydroxyde d'aluminium et du chlorure d'étain pentahydraté. 5,0 g d'hydroxyde d'aluminium sont soumis à une imprégnation à humidité naissante avec une solution aqueuse de chlorure d'étain (3,0g de $SnCl_4$, $5H_2O$ dans 4,5g d'eau). Le solide obtenu est ensuite séché à 80°C pendant 24 heures.

Ensuite, le solide est calciné sous débit d'air sec à la température de 700°C pendant 3 heures. A l'issue de ces traitements, l'oxyde d'étain déposé sur alumine et le support contient 15% poids d'étain. le catalyseur obtenu est mésoporeux.

La proportion de sites acides de Lewis du catalyseur C1 est supérieure à 90%.

Exemple 2 : Préparation d'un catalyseur C2 zéolithique Sn Beta (non conforme à l'invention).

Le catalyseur C2 est préparé selon l'enseignement du brevet US Patent 5,968,473.

Un gel de composition molaire : $SiO_2$:1/120$SnO_2$:0.54TEAOH:7.5$H_2O$: 0.54HF est préparé par hydrolyse du tétraéthylorthosilicate ou TEOS (98%,Merck) dans une solution aqueuse d'hydroxyde de tétraéthylammonium ou TEAOH (35%, Aldrich). Ensuite, une solution de $SnCl_4$, $5H_2O$ (98%, Aldrich) est ajoutée. Le mélange est agité jusqu'à évaporation totale de l'éthanol formé pendant l'hydrolyse du TEOS. Ensuite de l'acide fluorhydrique HF est ajouté (48%). Des germes de zéolithe beta désaluminés sont ainsi obtenus. La cristallisation est réalisée dans une autoclave agitée téflonée à 140°C. L'autoclave est ensuite refroidie. Le solide est récupéré par filtration, lavé abondamment avec de l'eau distillée. Après un séchage à 100°C, le solide est calciné à 580°C. Le solide est caractérisé par diffraction de rayons X : de la zéolithe Sn-beta est bien obtenue. Le catalyseur C2 est microporeux.

Exemple 3 : Transformation de la cellulose mettant en oeuvre les catalyseurs selon obtenus selon les exemples 1 et 2

Cet exemple concerne la conversion de la cellulose mettant en oeuvre les catalyseurs C1 et C2 pour la production d'acide lactique.

On introduit dans un autoclave de 100 mL 65 ml d'eau, 1,6g de cellulose Avicel® (70% cristallinité) et 0,68 g de catalyseur des exemples 1 et 2. On chauffe à 190°C et on injecte 5 MPa $H_2$. La pression totale atteint alors 6 MPa. Après 24h de réaction, le milieu réactionnel est prélevé et centrifugé. Le liquide réactionnel est ensuite analysé par chromatographie liquide sous haute pression (HPLC) en utilisant la réfractométrie pour déterminer la teneur en produits de conversion de la solution aqueuse selon les équations décrites ci-dessus.

La réaction de transformation a également été effectuée avec les supports d'oxydes $Al_2O_3$.

Les résultats obtenus sont référencés dans le tableau 1.

Tableau 1 : Conversion de cellulose et rendements en acide lactique et oligosaccharides et polymères solubles utilisant différents catalyseurs.

| catalyseur | conversion cellulose (%) | rendement molaire en acide lactique (%) | rendements molaires en oligosaccharides et polymères solubles (%) |
|---|---|---|---|
| $Al_2O_3$ (non conforme) | 35 | 3 | 7 |
| AlSn (C1) | 44 | 23 | 0 |
| Sn Beta (C2, non conforme) | 27 | 10 | 6 |

On trouve que pour le catalyseur Al$_2$O$_3$, non conforme à l'invention, la quantité d'acide lactique formé représente 3% en mole de la quantité de cellulose de départ, avec 7% en mole d'oligosaccharides et polymères solubles. La conversion de la cellulose est de 35%.

On trouve ainsi que pour le catalyseur AlSn, la quantité d'acide lactique formé représente 23% en mole de la quantité de cellulose de départ, sans formation d'oligosaccharides et polymères solubles. La conversion est de 44%.

L'utilisation de catalyseurs acide de Lewis zéolithique Sn beta conduit à la formation moins importante d'acide lactique formé. La quantité de produit acide lactique formé représente 10% en mole de la quantité de cellulose de départ. On note un formation de 6% d'oligosaccharides et de polymères solubles. La conversion est de 27%.

L'analyse du milieu réactionnel après 24h de réaction montre que le catalyseur C1 est stable dans les conditions de la réaction. En effet, la perte en étain est inférieure à 0.1%. Après, 24h de réaction, le catalyseur C2, non conformé à l'invention présente une perte en silicium de 10% et une perte en étain de 0.3% poids.

Ainsi, ces exemples démontrent l'obtention d'acide lactique à haut rendement par transformation directe de cellulose via des catalyseurs mésoporeux hétérogènes à base d'acide de Lewis Sn supportés sur des oxydes stables tout en limitant la formation d'oligosaccharides et de polymères solubles.

## Revendications

1. Procédé de transformation de biomasse cellulosique ou de cellulose en acide lactique comprenant la mise en contact de ladite biomasse ou cellulose en présence d'eau avec un catalyseur hétérogène non zéolithique à base d'étain et/ou antimoine, ledit catalyseur présentant des sites acides de type Lewis.

2. Procédé selon la revendication 1 dans lequel ledit catalyseur est à base d'oxyde d'étain et/ou d'antimoine.

3. Procédé selon l'une des revendications 1 ou 2 dans lequel ledit catalyseur est dispersé sur un support à base d'au moins un oxyde ou d'un support carboné.

4. Procédé selon la revendication 3 dans lequel ledit support à base d'oxyde est choisi parmi les oxydes d'aluminium et/ou de zirconium et/ou de titane et/ou de niobium.

5. Procédé selon la revendication 3 dans lequel le support carboné est choisi parmi les charbons actifs, le noir de carbone, les solides microporeux ou mésoporeux carbonés tels que les nanotubes de carbone ou les fibres de carbone.

6. Procédé selon l'une des revendications précédentes dans lequel la teneur en sites acides de type Lewis est supérieure à 50% de la teneur totale en sites acides.

7. Procédé selon l'une des revendications précédentes dans lequel la teneur en étain et/ou antimoine est comprise entre 1 et 100% poids, de préférence entre 1 et 50%, de préférence entre 1 et 30% poids et encore plus préférentiellement entre 1 et 20% poids par rapport à la masse totale du catalyseur.

8. Procédé selon l'une des revendications précédentes dans lequel les précurseurs d'étain ou d'antimoine sont choisis parmi les hydrures, les halogénures, les oxydes, les sulfures, ou des composés organométalliques respectivement d'étain ou d'antimoine.

9. Procédé selon l'une des revendication précédentes dans lequel le catalyseur est préparé par imprégnation d'une solution d'acide stannique et/ou d'oxyde d'antimoine et l'hydroxyde de zirconium et/ou de titane et/ou d'aluminium et/ou de niobium, éventuellement suivie d'un séchage.

10. Procédé selon l'une des revendications précédentes dans lequel le catalyseur subit au cours de sa préparation ou à l'issue de sa préparation un traitement thermique entre 300°C et 1000°C, sous air, sous atmosphère réductrice , ou sous azote, pur ou en mélange.

11. Procédé selon l'une des revendications précédentes dans lequel la transformation est mise en oeuvre dans un milieu contenant de l'eau, ledit milieu étant choisi parmi le groupe formé par un milieu liquide, tels que des alcools ou l'eau, un liquide ionique et un milieu supercritique de densité type liquide

12. Procédé selon l'une des revendications précédentes dans lequel la transformation est opérée à des températures comprises entre 160 et 250°C, de préférence entre 175 et 250°C, et à une pression comprise entre 0,5 et 20 MPa, de préférence entre 2 et 10 MPa.

13. Procédé selon l'une des revendications précédentes dans lequel le catalyseur est introduit à un rapport massique biomasse/catalyseur compris entre 1 et 1000, de préférence entre 1 et 500, très préférentiellement entre 1 et 50 et encore plus préférentiellement entre 1 et 25.

14. Procédé selon l'une des revendications précédentes dans lequel le taux de dilution de la biomasse dans le milieu aqueux est donc entre 1:1 et 1:1000, de préférence entre 1:1 et 1:500 et encore préférentiellement entre 1:5 et 1:100.

15. Procédé selon l'une des revendications précédentes dans lequel la réaction est mise en oeuvre en discontinu, ou en continu, dans un réacteur fermé ou semi-ouvert.

16. Procédé selon l'une des revendications précédentes **caractérisé en ce qu'**il est mis en oeuvre en continu, avec une vitesse massique horaire comprise entre 0,01 et 5 h$^{-1}$, de préférence entre 0,02 et 2 h$^{-1}$.


**Patentansprüche**

1. Verfahren zur Umwandlung von zellulosehaltiger Biomasse oder von Zellulose in Milchsäure, das das Inkontaktbringen der Biomasse oder der Zellulose in Wasser mit einem nicht-zeolithischen, heterogenen Katalysator auf der Basis von Zinn und/oder Antimon umfasst, wobei der Katalysator Lewis-Säurezentren aufweist.

2. Verfahren nach Anspruch 1, bei dem der Katalysator auf von Zinnoxid und/oder Antimonoxid basiert.

3. Verfahren nach einem der Ansprüche 1 oder 2, bei dem der Katalysator an einem Träger auf Basis mindestens eines Oxids oder eines Kohlenstoffträgers dispergiert ist.

4. Verfahren nach Anspruch 3, bei dem der Träger auf Basis eines Oxids ausgewählt ist aus Aluminium- und/oder Zirkon- und/oder Titan- und/oder Nioboxiden.

5. Verfahren nach Anspruch 3, bei dem der Kohlenstoffträger ausgewählt ist aus Aktivkohle, Ruß, mikroporösen oder mesoporösen kohlenstoffhaltigen Feststoffen, wie Kohlenstoff-Nanoröhrchen oder Kohlenstofffasern.

6. Verfahren nach einem der vorhergehenden Ansprüche, bei dem der Gehalt an Lewis-Säurezentren größer als 50% der Gesamtgehalt an Säurezentren ist.

7. Verfahren nach einem der vorhergehenden Ansprüche, bei dem die Menge an Zinn und/oder Antimon zwischen 1 und 100 Gew.-%, vorzugsweise zwischen 1 und 50 Gew.-%, vorzugsweise zwischen 1 und 30 Gew.-% und noch bevorzugter zwischen 1 und 20 Gew.-% in Bezug auf die Gesamtmasse des Katalysators liegt.

8. Verfahren nach einem der vorhergehenden Ansprüche, bei dem die Zinn- oder Antimonpräkursoren ausgewählt sind aus Hydriden, Halogeniden, Oxiden, Sulfiden oder organometallischen Zusammensetzungen jeweils des Zinns oder Antimons.

9. Verfahren nach einem der vorhergehenden Ansprüche, bei dem der Katalysator durch Imprägnierung einer Zinnsäurelösung und/oder Antimonoxidlösung und Zirkonhydroxid und/oder einer Titan- und/oder Aluminium- und/oder Nioblösung, gegebenenfalls gefolgt von einer Trocknung hergestellt wird.

10. Verfahren nach einem der vorhergehenden Ansprüche, bei dem der Katalysator während seiner Herstellung oder am Ende seiner Herstellung einer Wärmebehandlung zwischen 300°C und 1000°C bei Luft, bei reduzierender Atmosphäre oder bei Stickstoff, allein oder als Mischung, unterworfen wird.

11. Verfahren nach einem der vorhergehenden Ansprüche, bei dem die Umwandlung in einem wasserhaltigen Medium durchgeführt wird, wobei das Medium ausgewählt ist aus der Gruppe, die von einem flüssigen Medium, wie Alkohol oder Wasser, einer ionischen Flüssigkeit oder einem superkritischen Medium einer Dichte nach Art einer Flüssigkeit gebildet wird.

**12.** Verfahren nach einem der vorhergehenden Ansprüche, bei dem die Umwandlung bei Temperaturen zwischen 160 und 250°C, vorzugsweise zwischen 175 und 250°C und bei einem Druck zwischen 0,5 und 20 MPa, vorzugsweise zwischen 2 und 10 MPa durchgeführt wird.

**13.** Verfahren nach einem der vorhergehenden Ansprüche, bei dem der Katalysator bei einem Massenverhältnis Biomasse/Katalysator zwischen 1 und 1000, vorzugsweise zwischen 1 und 500, sehr bevorzugt zwischen 1 und 50 und noch bevorzugter zwischen 1 und 25 eingeführt wird.

**14.** Verfahren nach einem der vorhergehenden Ansprüche, bei dem das Verdünnungsverhältnis der Biomasse in dem wässrigen Medium somit zwischen 1:1 und 1:1000, vorzugsweise zwischen 1:1 und 1:500 und noch bevorzugter zwischen 1:5 und 1:100 liegt.

**15.** Verfahren nach einem der vorhergehenden Ansprüche, bei dem die Reaktion diskontinuierlich oder kontinuierlich in einem geschlossenen oder halboffenen Reaktor durchgeführt wird.

**16.** Verfahren nach einem der vorhergehenden Ansprüche, **dadurch gekennzeichnet, dass** es kontinuierlich mit einer Massenstundengeschwindigkeit zwischen 0,01 und 5 $h^{-1}$, vorzugsweise zwischen 0,02 und 2 $h^{-1}$ durchgeführt wird.

**Claims**

**1.** Process for converting cellulosic biomass or cellulose into lactic acid, comprising bringing said biomass or cellulose into contact, in the presence of water, with a non-zeolitic heterogeneous catalyst based on tin and/or antimony, said catalyst having Lewis-type acid sites.

**2.** Process according to claim 1, wherein said catalyst is based on tin oxide and/or antimony oxide.

**3.** Process according to either claim 1 or claim 2, wherein said catalyst is dispersed on a support based on at least one oxide or on a carbon-containing support.

**4.** Process according to claim 3, wherein said support based on oxide is chosen from the oxides of aluminium and/or of zirconium and/or of titanium and/or of niobium.

**5.** Process according to claim 3, wherein the carbon-containing support is chosen from activated carbons, carbon black, carbon-containing microporous or mesoporous solids such as carbon nanotubes or carbon fibres.

**6.** Process according to any one of the preceding claims, wherein the content of Lewis-type acid sites is greater than 50% of the total content of acid sites.

**7.** Process according to any one of the preceding claims, wherein the content of tin and/or antimony is between 1 and 100% by weight, preferably between 1 and 50%, preferably between 1 and 30% by weight and yet more preferably between 1 and 20% by weight, based on the total mass of the catalyst.

**8.** Process according to any one of the preceding claims, wherein the precursors of tin or antimony are chosen from the hydrides, halides, oxides, sulfides, or organometallic compounds of tin or antimony, respectively.

**9.** Process according to any one of the preceding claims, wherein the catalyst is prepared by impregnation of a solution of stannic acid and/or of antimony oxide and zirconium hydroxide and/or titanium hydroxide and/or aluminium hydroxide and/or niobium hydroxide, optionally followed by drying.

**10.** Process according to any one of the preceding claims, wherein the catalyst undergoes, during its preparation or at the end of its preparation, a heat treatment at between 300°C and 1000°C, in air, under a reducing atmosphere, or under nitrogen, pure or in a mixture.

**11.** Process according to any one of the preceding claims, wherein the conversion is carried out in a medium containing water, said medium being chosen from the group formed of a liquid medium, such as alcohols or water, an ionic liquid and a supercritical medium of liquid-type density.

12. Process according to any one of the preceding claims, wherein the conversion is carried out at temperatures of between 160 and 250°C, preferably between 175 and 250°C, and at a pressure of between 0.5 and 20 MPa, preferably between 2 and 10 MPa.

13. Process according to any one of the preceding claims, wherein the catalyst is introduced in a mass ratio biomass/catalyst of between 1 and 1000, preferably between 1 and 500, very preferably between 1 and 50 and yet more preferably between 1 and 25.

14. Process according to any one of the preceding claims, wherein the rate of dilution of the biomass in the aqueous medium is therefore between 1:1 and 1:1000, preferably between 1:1 and 1:500 and yet more preferably between 1:5 and 1:100.

15. Process according to any one of the preceding claims, wherein the reaction is carried out discontinuously or continuously, in a closed or semi-open reactor.

16. Process according to any one of the preceding claims, **characterised in that** it is carried out continuously, at a mass hourly velocity of between 0.01 and 5 $h^{-1}$, preferably between 0.02 and 2 $h^{-1}$.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- EP 2011569 A
- WO 03035582 A
- US 5968473 A

**Littérature non-brevet citée dans la description**

- **BRØNSTED.** *Angew. Chem. Int. Ed.,* 2008, vol. 47, 8047-8050
- *Catal. Lett.,* 2009, vol. 133, 221-226
- *Science,* 2010, vol. 328, 602-605
- **FANGMIN JIN ; HEIJI EINOMOTO.** *J. Mater. Sci.,* 2008, vol. 43, 2463-2471
- **KONG et al.** *J. Chem. Technol. Biotechnol.,* 2008, vol. 83, 383-388
- **SHIMIZU et al.** *Green Chem.,* 2009, vol. 11, 1627-1632
- **ZHANG et al.** *Angew. Chem. Int. Ed.,* 2008, vol. 47, 8510-8513
- **M. GUISNET ; P. AYRAULT ; C. COUTANCEAU ; M.F. ALVAREZ ; J. DATKA.** *J. Chem. Soc., Faraday Trans.,* 1997, vol. 93, 1661